# FASCICULE DE BREVET EUROPEEN

(11) **EP 3 849 472 B1**
(45) Date de publication et mention de la délivrance du brevet: **29.06.2022**
(21) Numéro de dépôt: 19769760.0
(22) Date de dépôt: 13.09.2019
(51) Int. Cl.: A61F 2/24

(54) **IMPLANT POUR VALVE CARDIAQUE**
HERZKLAPPENIMPLANTAT
HEART VALVE IMPLANT

(30) Priorité: 13.09.2018 FR 1858233
(43) Date de publication de la demande: 21.07.2021
(73) Titulaire: Université de Technologie de Compiègne, 60200 Compiègne (FR); Université Paris-Est Créteil Val de Marne, 94010 Creteil Cedex (FR); ASSISTANCE PUBLIQUE, HOPITAUX DE PARIS, 75004 Paris (FR); INSTITUT NATIONAL DE LA SANTE ET DE LA RECHERCHE MEDICALE - INSERM, 75013 Paris (FR); CENTRE NATIONAL DE LA RECHERCHE SCIENTIFIQUE (CNRS), 75016 Paris (FR)
(72) Inventeur: SALSAC, Anne-Virginie, 60750 CHOISY-AU-BAC (FR); LAPERROUSAZ, Adrien, 74550 PERRIGNIER (FR); BERGÖEND, Eric, 94100 SAINT MAUR DES FOSSES (FR); COUËTIL, Jean-Paul, 92420 VAUCRESSON (FR)
(74) Mandataire: Regimbeau
(86) Numéro de dépôt international: PCT/EP2019/074477
(87) Numéro de publication internationale: WO 2020/053384

(56) Documents cités:
- WO-A1-2007/078772
- WO-A1-2016/099650
- CN-U- 202 859 228

## Description

### DOMAINE TECHNIQUE GENERAL

La présente invention concerne un implant cardiaque destiné au traitement de fuites valvulaires. Il est particulièrement adapté pour le traitement de l'insuffisance mitrale chez un patient, mais son utilisation pourra être étendue aux autres valves cardiaques outre la valve mitrale. Plus précisément, l'invention concerne un implant pouvant être implanté par voie percutanée et apposé sur un des feuillets de la valve cardiaque à traiter.

### ETAT DE L'ART

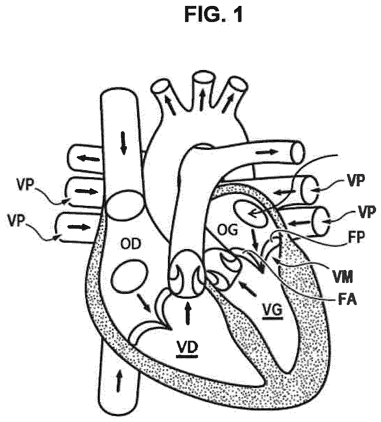

La figure 1 illustre le cœur d'un patient. Il comprend un ventricule gauche VG séparé de l'oreillette gauche OG par la valve mitrale VM. Lorsque le ventricule gauche VG se contracte (systole), l'oreillette gauche OG se remplit de sang oxygéné provenant des veines pulmonaires VP, la valve mitrale VM étant alors fermée. Puis, le ventricule gauche VG se relâche (diastole). La valve mitrale VM s'ouvre et le sang passe de l'oreillette gauche OG vers le ventricule gauche VG. Puis celui-ci se contracte, ouvrant la valve aortique VA et fermant la valve mitrale VM, afin d'éjecter le sang oxygéné dans l'aorte A, vers le reste de l'organisme.

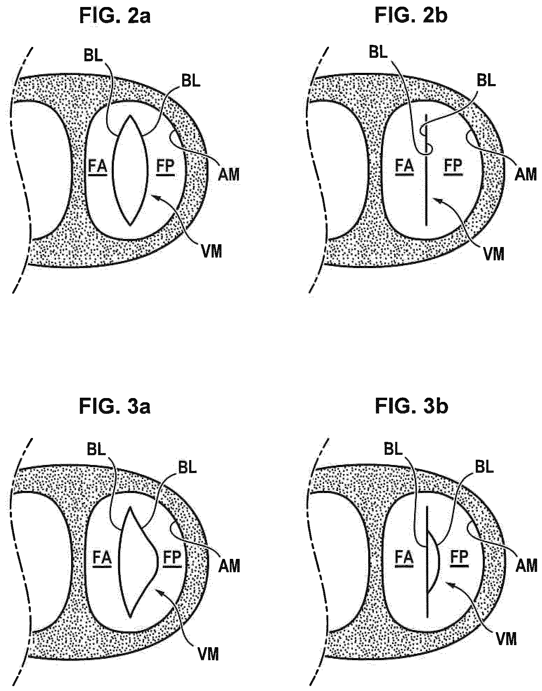

Comme illustré sur les figures 2a et 2b, la valve mitrale VM est constituée d'un anneau mitral AM qui délimite l'ouverture entre l'oreillette gauche OG et le ventricule gauche VG, d'un feuillet postérieur FP et d'un feuillet antérieur FA. Le feuillet postérieur FP et le feuillet antérieur FA s'étendent respectivement depuis la partie postérieure et depuis la partie antérieure de l'anneau mitral AM. L'Appareil Sous-Valvulaire Mitral, ASVM, est formé de cordages tendineux reliant les feuillets postérieur FP et antérieur FA à la paroi du ventricule gauche VG par l'intermédiaire des muscles papillaires.

Les figures 2a et 2b illustrent respectivement, en vue de dessus, une valve mitrale VM saine lors de la diastole et lors de la systole. Lors de la diastole, les feuillets antérieur et postérieur FA et FP de la valve mitrale VM s'écartent l'un de l'autre, permettant au sang de s'écouler de l'oreillette gauche OG vers le ventricule gauche VG. Lors de la systole, les feuillets antérieur et postérieur FA et FP de la valve mitrale VM viennent en contact l'un avec l'autre, de manière à fermer le passage entre le ventricule gauche VG et l'oreillette gauche OG, assurant ainsi la coaptation et empêchant tout reflux de sang depuis le ventricule gauche VG vers l'oreillette gauche OG.

Les figures 3a et 3b illustrent, en vue de dessus, une valve mitrale VM d'un patient souffrant d'insuffisance mitrale, respectivement lors de la diastole (mouvement de relaxation du cœur) et lors de la systole (mouvement de contraction du cœur). On remarque sur la figure 3b qu'une partie du feuillet postérieur FP n'est pas en mesure de venir en contact avec le feuillet antérieur FA lors de la systole. La valve mitrale VM ne se ferme pas correctement, ce qui provoque une régurgitation - un reflux - de sang depuis le ventricule gauche VG vers l'oreillette gauche OG. Le flux sanguin envoyé par le cœur vers le reste du corps s'en trouve diminué.

Une telle insuffisance, appelée insuffisance mitrale, s'accompagne généralement d'une dilatation du ventricule gauche VG. Cette dilatation entraîne une augmentation du diamètre de l'anneau mitral AM, ce qui provoque un écartement encore plus important entre les feuillets postérieur FP et antérieur FA lors de la systole, et donc accroît encore l'insuffisance mitrale (réalisant ainsi un cercle vicieux).

En dehors du traitement médical à visée cardiaque, le traitement de référence de cette pathologie consiste en une réparation de la valve, ou un remplacement par voie chirurgicale. Lorsque cette pathologie est secondaire à une myocardiopathie (insuffisance mitrale secondaire ou fonctionnelle), une intervention chirurgicale présente un risque non négligeable, du fait de la myocardiopathie associée, d'autres problèmes de santé associés ou de l'âge avancé du patient.

Plusieurs solutions percutanées pour traiter l'insuffisance mitrale ont ainsi été proposées afin de limiter le caractère invasif de l'intervention.

Le document US 2003/0130571 décrit un système de réparation de la valve mitrale au moyen duquel les bords libres des feuillets antérieur et postérieur sont agrafés ensemble au niveau de leur partie centrale afin d'assurer la fermeture complète de la valve mitrale lors de la systole. Lors de la diastole, l'ouverture formée entre les feuillets antérieur et postérieur agrafés en leur milieu présente la forme d'un nœud papillon.

Pour réparer de la sorte la valve mitrale, le système proposé dans ce document comprend un guide, une tige creuse munie de mâchoires, un cathéter à l'extrémité duquel s'étend un ballon gonflable, et une agrafe. Le guide est d'abord inséré dans le ventricule gauche jusqu'à l'oreillette gauche par l'intermédiaire d'une valve ménagée préalablement dans la paroi du ventricule gauche au niveau de l'apex de ce dernier. Puis, la tige est glissée le long du guide jusqu'à ce que les mâchoires soient disposées dans l'oreillette gauche. Le guide est ensuite retiré et les mâchoires ouvertes, dans l'oreillette gauche, de sorte à disposer les bords libres des feuillets antérieur et postérieur en regard de l'apex du ventricule gauche et les maintenir dans cette position dite position de prise. Le cathéter est alors glissé à l'intérieur de la tige, jusqu'à ce que le ballon soit disposé dans l'oreillette gauche où il est gonflé. Les mâchoires sont ensuite glissées dans le ventricule gauche, tandis que le ballon maintient les bords libres des feuillets antérieur et postérieur dans la position de prise, et les mâchoires viennent enserrer lesdits bords libres. Enfin, l'agrafe est acheminée, le long de l'intérieur de la tige, vers les bords libres des feuillets antérieur et postérieur enserrés par les mâchoires, jusqu'à les fixer ensemble au niveau de leur partie centrale.

Une telle réparation de la valve mitrale, notamment la pose de l'implant, nécessite une intervention chirurgicale incluant une thoracotomie, ce qui augmente les risques de la procédure.

Une autre solution est proposée dans le document WO 2008/141322. Ce document décrit un implant pour valve mitrale pouvant être implanté par voie trans-apicale. Cet implant comprend une tige à une première extrémité de laquelle s'étend une portion d'ancrage destinée à être insérée dans l'apex du myocarde et, à une deuxième extrémité de laquelle se déploie un ballonnet destiné à venir en contact avec les feuillets antérieur et postérieur de la valve mitrale, lors de la systole, évitant ainsi tout reflux de sang entre le ventricule gauche et l'oreillette gauche. Ce dispositif augmente la surface de coaptation entre les feuillets valvulaires. Pour cela, le ballonnet coulisse librement sur la tige. Lors de la systole, la différence de pression entre le ventricule gauche VG et l'oreillette gauche OG fait remonter le ballonnet vers l'oreillette gauche OG. Il se positionne ainsi entre les deux feuillets valvulaires et diminue la régurgitation valvulaire en systole. Le ballonnet est gonflable, ce qui permet son implantation par voie percutanée dans un état plié. Le ballonnet est déployé une fois l'implant correctement positionné dans le cœur du patient.

Cependant, en l'état actuel, cette solution ne peut être mise en œuvre que par abord trans-thoracique et non par voie percutanée.

Le document US 2014/0236198 propose en outre une solution alternative à celle décrite dans le document US 2003/0130571, dans laquelle les bords libres des feuillets antérieur et postérieur de la valve mitrale sont également agrafés ensemble au niveau de leur partie centrale, de sorte que, lors de la diastole, l'ouverture formée entre lesdits feuillets antérieur et postérieur présente la forme d'un nœud papillon. Dans ce document cependant, la réparation de la valve mitrale peut être réalisée par voie percutanée. Pour cela, le dispositif proposé comprend une tige à l'extrémité de laquelle une agrafe est fixée de manière amovible. L'agrafe comprend une paire de bras distaux et une paire de bras proximaux, les bras de chacune des paires étant disposés de manière radialement opposée. Lors de l'insertion du dispositif dans le système vasculaire du patient, les bras de chaque paire de l'agrafe sont repliés et s'étendent le long de la tige. Une fois l'agrafe acheminée jusqu'à la valve mitrale, les bras sont déployés de sorte qu'un premier couple de bras proximal et distal enserre le feuillet antérieur, tandis que le deuxième couple de bras proximal et distal enserre le feuillet postérieur, fixant ainsi ensemble les feuillets antérieur et postérieur au niveau de leur partie centrale. La tige et l'agrafe peuvent ensuite être découplées et la tige retirée.

Comparativement à la solution proposée dans le document US 2003/0130571, cette solution présente l'avantage de permettre une mise en œuvre par voie percutanée, limitant ainsi les risques de la procédure.

Cependant, comme pour la solution proposée dans le document US 2003/0130571, la calcification des feuillets antérieur et postérieur limite l'efficacité d'un tel agrafage des feuillets de la valve mitrale.

Il a également été proposé dans le document US 9,510,948 un implant comprenant une mâchoire susceptible d'enserrer un feuillet de valve cardiaque. Pour amener cet implant à proximité de la valve cardiaque, un dispositif de largage distinct de l'implant (illustré sur la figure 12A du US 9,510,948) est utilisé. Le dispositif de largage comprend une tige, et une deuxième mâchoire montée sur la tige (distincte de la mâchoire de l'implant lui-même). L'implant est enserré par les mors de la deuxième mâchoire. Une fois l'implant à proximité, l'implant est dégagé hors de la deuxième mâchoire. Toutefois, cet implant présente l'inconvénient d'être difficile à poser en manœuvrant la tige à distance. En particulier, le dégagement de l'implant hors de la mâchoire du dispositif de largage est difficile à réaliser.

Il a également été proposé dans le document WO2007/078772 un appareil permettant d'aider au traitement de la régurgitation de sang à travers une valve cardiaque. L'implant proposé comporte un élément d'occlusion, notamment un ballonnet, configuré pour être placé dans la valve mitrale, de telle sorte qu'au moins une partie de l'élément d'occlusion soit à proximité de la valve cardiaque. Un fil de suspension permet un positionnement de l'élément d'occlusion dans la valve cardiaque. Le fil de suspension comprend une partie de fixation pour fixer le fil de suspension à une paroi cardiaque entourant la chambre cardiaque contenant la valve cardiaque. Le ballonnet est alors situé entre les deux feuillets. Toutefois, la fixation de ce fil de suspension par l'élément de fixation n'est pas manipulable de façon triviale, et l'élément d'occlusion; le ballonnet, n'est pas facilement positionnable à l'endroit exact où se trouve la régurgitation de sang. L'efficacité de ce dispositif en est donc limitée.

Par ailleurs, on connait le document US 14,577,852. Ce document enseigne un implant, notamment pour valve mitrale, utilisé dans le traitement de l'insuffisance mitrale. Plus précisément, ce document décrit un clip mitral, servant à rapprocher les deux feuillets de la valve mitrale, lorsque l'implant est fixé sur le premier feuillet de ladite valve. Toutefois, le rapprochement des deux feuillets peut parfois ne pas être suffisant pour éviter toute régurgitation de sang. WO

2016/099640 A1 décrit un mécanisme unidirectionnel qui permet aux tissus d'entrer dans le mécanisme mais de ne pas en sortir facilement, tel qu'un ressort à lames, une saillie, un bras pivotant et un ou plusieurs éléments de frottement.

Il existe donc un besoin de proposer un implant pour valve mitrale permettant d'obtenir une réparation efficace de la valve mitrale et pouvant être implanté avec un minimum de risques pour le patient et d'une manière plus aisée.

### PRESENTATION DE L'INVENTION

Plus précisément, la présente invention répond à ce problème en proposant, selon un premier aspect, un implant pour une valve cardiaque comprenant deux feuillets, notamment une valve mitrale, l'implant comprenant :
- une mâchoire comprenant un corps et un mors articulé en rotation avec le corps pour pivoter entre une position ouverte et une position fermée dans laquelle le corps et le mors sont aptes à enserrer un premier feuillet parmi les feuillets de la valve cardiaque, de sorte à fixer l'implant sur le premier feuillet,
- un dispositif de déplacement comprenant un élément poussoir, le dispositif de déplacement étant configuré pour déplacer le mors par rapport au corps depuis l'une parmi des positions ouverte et fermée à l'autre position, lorsque l'élément poussoir est poussé par une tige,
- un ballonnet fixé à la mâchoire et configuré pour obturer au moins en partie une portion d'ouverture subsistant entre les feuillets de la valve cardiaque lors d'une systole, lorsque l'implant est fixé sur le premier feuillet, de sorte à limiter un reflux de sang à travers ladite portion d'ouverture subsistant lors de la systole.

L'implant selon le premier aspect de l'invention peut comprendre en outre les caractéristiques optionnelles suivantes, prises seules ou en combinaison lorsque cela est techniquement possible.

De préférence, l'implant est configuré pour être inséré dans un cathéter principal 100, de sorte à être implanté par voie percutanée ou par voie trans-apicale.

De préférence, le ballonnet est gonflable.

De préférence, le ballonnet présente une forme globalement allongée s'étendant suivant un axe transversal parallèle au premier axe de rotation.

De préférence, le ballonnet présente une première face agencée en regard de la mâchoire, la première face étant convexe entre chacune des extrémités du ballonnet suivant l'axe transversal.

De préférence, l'implant comprend une languette de support fixée au ballonnet, ladite languette de support comprenant un matériau à mémoire de forme présentant dans une première gamme de températures, un premier état de forme dans lequel la languette de support est repliée et, dans une deuxième gamme de températures, un deuxième état de forme dans lequel la languette de support est déployée.

De préférence, la mâchoire comprend un dispositif de verrouillage configuré pour verrouiller la mâchoire en position fermée, et le dispositif de déplacement est configuré pour agir sur le dispositif de verrouillage pour que le mors soit verrouillé en position fermée, lorsque le dispositif de déplacement déplace le mors par rapport au corps dans la position fermée.

De préférence, le dispositif de verrouillage comprend une genouillère.

De préférence, le mors est articulé en rotation autour d'un premier axe de rotation par rapport au corps, et dans lequel la mâchoire comprend une bielle articulée en rotation avec le mors autour d'un deuxième axe de rotation, ledit deuxième axe de rotation étant parallèle avec le premier axe de rotation, dans lequel la mâchoire comprend au moins un élément de bascule articulé en rotation avec la bielle autour d'un troisième axe de rotation d'une part et avec le corps de la mâchoire autour d'un quatrième axe de rotation d'autre part, les troisième et quatrième axes de rotation étant parallèles entre eux et avec les premier et deuxième axes de rotation, et dans lequel l'élément de bascule et la bielle forment ensemble la genouillère, dans lequel la genouillère est configurée pour se déplacer entre une configuration déverrouillée dans laquelle le troisième axe de rotation est situé d'un premier côté du plan formé par les deuxième et quatrième axes de rotation et la mâchoire est en position ouverte, et une configuration verrouillée dans laquelle le troisième axe de rotation est situé d'un deuxième côté dudit plan et la mâchoire est en position fermée.

De préférence, la mâchoire comprend un dispositif de blocage configuré pour bloquer la genouillère en configuration verrouillée.

De préférence, le dispositif de blocage comprend
- une butée de la bielle configurée pour venir en contact avec le ou les éléments de bascule, lorsque la genouillère est en configuration verrouillée, ou
- une protubérance du mors configurée pour venir en contact avec la bielle, lorsque la genouillère est en configuration verrouillée.

De préférence, le corps s'étend le long d'un axe longitudinal, le mors est articulé en rotation par rapport au corps autour d'un premier axe de rotation perpendiculaire à l'axe longitudinal, et l'élément poussoir est configuré pour se déplacer en translation suivant l'axe longitudinal par rapport au corps de la mâchoire.

De préférence, le dispositif de déplacement comprend par ailleurs un élément de levier articulé en rotation par rapport à la bielle autour du troisième axe de rotation d'une part et par rapport à l'élément poussoir autour d'un cinquième axe de rotation parallèle avec le premier axe de rotation.

De préférence, le corps et le mors comprennent chacun une partie de prise agencées en regard l'une de l'autre et configurées pour enserrer le premier feuillet de la valve cardiaque, par exemple le feuillet postérieur d'une valve mitrale, lorsque la mâchoire est en position fermée, et dans lequel l'élément poussoir est en outre configuré pour se déplacer en translation suivant l'axe longitudinal par glissement contre une face du corps, opposée à la partie de prise du mors.

De préférence, le corps s'étend le long d'un axe longitudinal, et l'élément poussoir est pourvu d'une ouverture s'étendant suivant l'axe longitudinal et configurée pour coopérer avec l'extrémité distale de la tige.

De préférence, le corps comprend un élément de guidage configuré pour guider une translation de l'extrémité distale de la tige suivant l'axe longitudinal.

De préférence, l'élément de guidage s'étend depuis la face du corps opposée à la partie de prise du mors et comprend une ouverture coaxiale avec et en regard de l'ouverture de l'élément poussoir, ladite ouverture de l'élément de guidage étant configurée pour accueillir de manière coulissante l'extrémité distale de la tige.

De préférence, le corps et le mors comprennent chacun une partie de prise agencée en regard l'une de l'autre et configurée pour enserrer le premier feuillet de la valve cardiaque, par exemple le feuillet postérieur d'une valve mitrale, lorsque la mâchoire est en position fermée, et dans lequel la partie de prise du corps ou du mors comprend une pluralité de pointes configurées pour se planter dans le premier feuillet, lorsque la mâchoire enserre le premier feuillet.

De préférence, au moins une ouverture est ménagée dans la partie de prise du mors ou du corps, en regard des pointes du corps.

Il est également proposé, selon un deuxième aspect de l'invention, un ensemble comprenant l'implant pour une valve cardiaque selon le premier aspect de l'invention et une tige dont l'extrémité distale est configurée pour coopérer avec l'élément poussoir de l'implant, de sorte à ouvrir et à fermer ladite mâchoire.

De préférence, cet ensemble comprend en outre un cathéter principal configuré pour accueillir l'implant et la tige.

### PRESENTATION DES FIGURES

D'autres caractéristiques, buts et avantages de l'invention ressortiront de la description qui suit, qui est purement illustrative et non limitative, et qui doit être lue en regard des dessins annexés, sur lesquels :
- la figure 1 (déjà décrite) est une vue en coupe sagittale et schématique du cœur d'un patient, montrant le positionnement d'une valve mitrale et de trois autres valves cardiaques ;
- les figures 2a et 2b (déjà décrites) sont des vues de dessus d'une valve mitrale saine d'un patient, respectivement lors de la diastole et lors de la systole ;
- les figures 3a et 3b (déjà décrites) sont des vues de dessus d'une valve mitrale d'un patient souffrant d'insuffisance mitrale, respectivement lors de la diastole et lors de la systole ;
- la figure 4 est une vue en perspective d'un implant pour la valve mitrale d'un patient souffrant d'insuffisance mitrale selon un mode de réalisation de l'invention ;
- la figure 5a est une vue en perspective d'un exemple de ballonnet de l'implant illustré à la figure 4 ;
- les figures 5b à 5e sont des vues en perspectives illustrant d'autres exemples de ballonnets pouvant faire partie de l'implant illustré sur la figure 4 ;

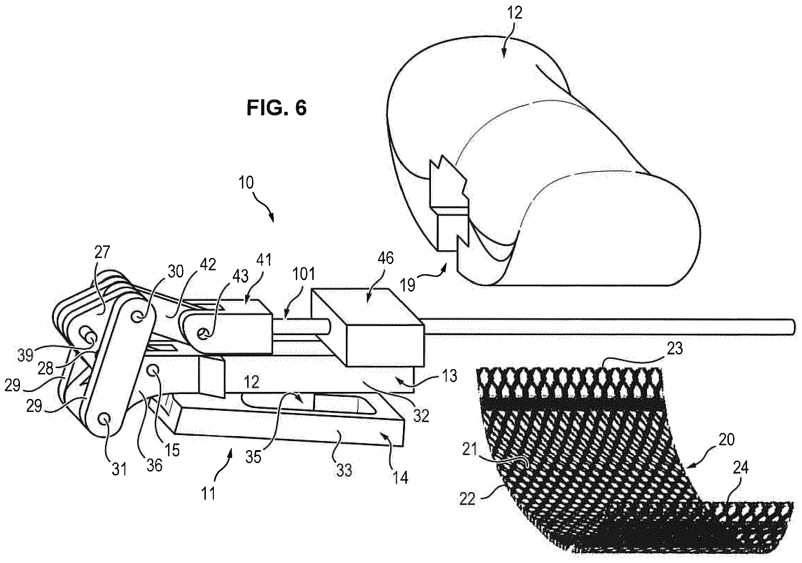

- la figure 6 est une vue en perspective éclatée de l'implant illustré à la figure 4 ;
- la figure 7 représente de manière schématique une étape de gonflage du ballonnet ;
- la figure 8 est une vue en perspective d'un exemple de mâchoire pour l'implant illustré à la figure 4, dans une position ouverte ;
- la figure 9 est une vue de profil de la mâchoire illustrée à la figure 8, dans une position intermédiaire ;
- la figure 10a est une vue de profil de la mâchoire illustrée aux figures 8 et 9, dans une position fermée ;
- la figure 10b est une vue en coupe longitudinale et en perspective de la mâchoire illustrée à la figure 10a ;
- la figure 11 est une vue de profil d'un autre exemple de mâchoire que celui illustré aux figures 8 à 10a-b, dans la position fermée ;
- la figure 12 est un ordinogramme d'un procédé de pose de l'implant illustré à la figure 4, par voie trans-septale, selon un mode de réalisation de l'invention ;
- la figure 13 est une vue en coupe sagittale, schématique, du côté gauche du cœur d'un patient lors d'une première étape du procédé illustré à la figure 12 ;
- la figure 14 est une vue en coupe sagittale, schématique, du côté gauche du cœur d'un patient lors d'une deuxième étape du procédé illustré à la figure 12 ;
- la figure 15 est une vue en coupe sagittale, schématique, du côté gauche du cœur d'un patient lors d'une quatrième étape du procédé illustré à la figure 12 ;
- la figure 16 est une vue en coupe sagittale, schématique, du côté gauche du cœur d'un patient lors d'une sixième étape du procédé illustré à la figure 12 ;
- la figure 17 est une vue en coupe sagittale, schématique, du côté gauche du cœur d'un patient lors d'une septième étape du procédé illustré à la figure 12 ;
- la figure 18 est une vue en coupe sagittale, schématique, du côté gauche du cœur d'un patient lors d'une huitième étape du procédé illustré à la figure 12 ;
- la figure 19 est une vue en coupe sagittale, schématique, du côté gauche du cœur d'un patient lors d'une neuvième étape du procédé illustré à la figure 12 ;
- la figure 20 est une vue en coupe sagittale, schématique, du côté gauche du cœur d'un patient lors d'une dixième étape du procédé illustré à la figure 12 ;
- la figure 21 est une vue en coupe sagittale, schématique, du côté gauche du cœur d'un patient lors d'une onzième étape du procédé illustré à la figure 12 ;
- la figure 22 est une vue en coupe sagittale, schématique, du côté gauche du cœur d'un patient pendant la systole, lors d'une douzième étape du procédé illustré à la figure 12 ;
- la figure 23 est une vue en coupe sagittale, schématique, du côté gauche du cœur d'un patient lors d'une quatorzième étape du procédé illustré à la figure 12 ;
- la figure 24 est une vue en coupe sagittale, schématique, du côté gauche du cœur d'un patient pendant la systole, lors d'une quinzième étape du procédé illustré à la figure 12 ;
- la figure 25 est une vue en coupe sagittale, schématique, du côté gauche du cœur d'un patient pendant la systole, lors d'une seizième étape du procédé illustré à la figure 12 ;

### DESCRIPTION DETAILLEE D'UN MODE DE REALISATION

Comme décrit précédemment, les figures 3a et 3b illustrent la valve mitrale VM d'un patient souffrant d'insuffisance mitrale, respectivement lors de la diastole et lors de la systole. Lors de la diastole, les feuillets antérieur et postérieur FA et FP de la valve mitrale VM délimitent entre eux une ouverture O permettant une circulation du sang de l'oreillette gauche OG vers le ventricule gauche VG. La valve mitrale VM est alors ouverte. Lors de la systole, les feuillets antérieur et postérieur FA et FP de la valve mitrale VM ne viennent qu'en partie au contact l'un de l'autre, de sorte qu'une portion O1 de l'ouverture O subsiste. La valve mitrale VM n'est donc que partiellement fermée, provoquant un reflux de sang depuis le ventricule gauche VG vers l'oreillette gauche OG.

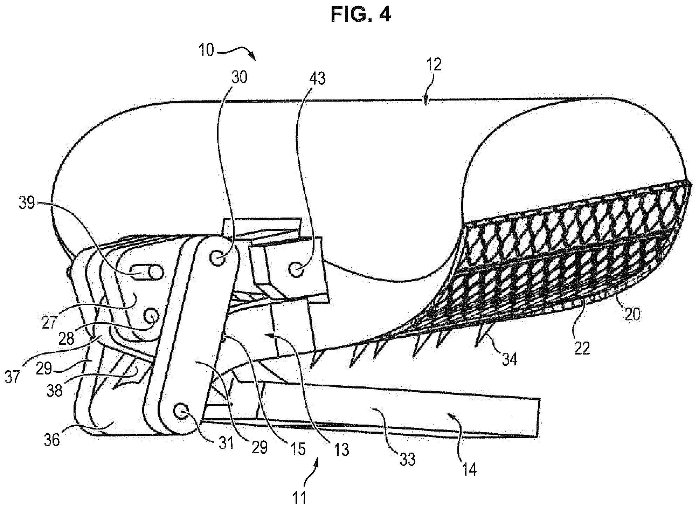

La figure 4 représente de manière schématique un implant 10 pour une valve mitrale VM d'un patient souffrant d'insuffisance mitrale selon un mode de réalisation possible de l'invention.

L'implant 10 comprend une mâchoire 11 et un ballonnet 12 fixé sur ladite mâchoire 11.

La mâchoire 11 comprend un corps 13 s'étendant suivant un axe longitudinal X et un mors 14 articulés en rotation par rapport au corps 13 autour d'un premier axe de rotation 15 perpendiculaire à l'axe longitudinal X. Le mors 14 de la mâchoire 11 est configuré pour pivoter autour du premier axe de rotation 15 par rapport au corps 13 entre une position ouverte et une position fermée dans laquelle le corps 13 et le mors 14 de la mâchoire 11 sont aptes à enserrer un des feuillets (par exemple le feuillet postérieur FP) de la valve mitrale VM, fixant ainsi l'implant 10 sur ledit feuillet.

Le ballonnet 12 est configuré pour obturer au moins en partie la portion O1 d'ouverture subsistant entre les feuillets postérieur FP et antérieur FA de la valve mitrale VM lors de la systole, après que l'implant 10 a été fixé sur ledit feuillet, de sorte à limiter le reflux de sang s'opérant à travers la portion O1 d'ouverture de la valve mitrale VM lors de la systole.

L'implant est plus susceptible d'être fixé au feuillet postérieur FP, car la stabilité de l'implant sera probablement meilleure que s'il est fixé au feuillet antérieur FA.

L'implant 10 est configuré pour pouvoir être inséré dans un cathéter principal 100, de sorte à être implanté par voie percutanée ou par voie trans-thoracique. Lorsque l'implant 10 est implanté par voie percutanée, le cathéter principal est par exemple inséré dans la veine fémorale au niveau du pli de l'aine et remonté, par voie trans-septale (à travers le septum inter-atrial : membrane qui sépare l'oreillette gauche OG de l'oreillette droite OD) jusqu'à l'oreillette gauche OG puis jusqu'à la valve mitrale VM. Lorsque l'implant 10 est implanté par voie trans-thoracique, le cathéter principal 100 est inséré au niveau de la pointe du ventricule gauche VG.

La mâchoire 11 est configurée pour coopérer avec l'extrémité distale d'une tige d'actionnement 101 par l'intermédiaire de laquelle la mâchoire 11 est ouverte et fermée. La tige 101 est par exemple montée en partie à l'intérieur d'un cathéter de largage 102, l'extrémité distale de la tige 101 étant agencée à l'extérieur dudit cathéter de largage 102. Dans une première configuration, la tige 101 est solidaire du cathéter de largage 102 et dans une deuxième configuration, la tige 101 est libre de coulisser le long du cathéter de largage 102. Ainsi, dans la première configuration, la tige 101 ne peut pas coulisser à l'intérieur du cathéter de largage 102. La tige 101 et le cathéter de largage 102 se déplacent donc d'un seul tenant. En revanche, dans la deuxième configuration, la tige 101 peut effectuer des mouvements de va-et-vient en coulissant à l'intérieur du cathéter de largage 102.

De préférence, le ballonnet 12 est gonflable. De cette manière, lorsque l'implant 10 est déplacé le long du cathéter principal 100 jusqu'à la valve mitrale VM, le ballonnet 12 est à l'état dégonflé. Le diamètre du cathéter principal 100 peut ainsi être plus faible et le déplacement de l'implant 10 le long dudit cathéter principal 100 est simplifié. En revanche, une fois l'implant 10 fixé au feuillet postérieur FP de la valve mitrale VM, le ballonnet 12 peut être gonflé. En utilisation, le ballonnet demeure à l'état gonflé.

Le ballonnet 12 est par exemple gonflé par l'intermédiaire de la tige 101. Pour cela, le ballonnet 12 comprend par exemple une ouverture communiquant avec un espace intérieur dudit ballonnet 12. L'ouverture est configurée pour communiquer de manière fluidique avec une lumière ménagée dans la tige 101. Ainsi, un matériau de remplissage peut être injecté par l'intermédiaire de la lumière de la tige 101 et de l'ouverture du ballonnet 12 pour remplir l'espace intérieur dudit ballonnet 12 et le gonfler.

Dans un premier temps, avant l'injection du matériau de remplissage, il est possible d'injecter du sérum physiologique dans l'espace intérieur du ballonnet 12. Grâce à cette étape préalable, l'opérateur peut déterminer le volume de matériau de remplissage nécessaire pour que le ballonnet 12 à l'état gonflé obture suffisamment la portion O1 d'ouverture de la valve mitrale VM et donc que le reflux de sang soit diminué de façon significative voire supprimé.

Ainsi, après avoir évacué le sérum physiologique du ballonnet 12, l'opérateur peut remplir le ballonnet 12 avec le volume de remplissage déterminé du matériau de remplissage.

Dans un deuxième temps, le matériau de remplissage est injecté dans l'espace intérieur du ballonnet 12. Le matériau de remplissage est de préférence un matériau biocompatible présentant une masse volumique inférieure ou de l'ordre de celle du sang (de 1056 à 1066 kg/m³). Le matériau de remplissage est par exemple un matériau à base de résine auto-durcissante. Comme le matériau de remplissage est alors auto-durcissant, il évite tout risque de dégonflement ou de perte de pression à l'intérieur du ballonnet 12 après remplissage.

Alternativement, le matériau de remplissage peut être un matériau présentant une structure alvéolaire, tel qu'une mousse ou une éponge. D'autres matériaux possibles pour le remplissage sont : hydrogels à base d'acide hyaluronique, poly(ethylène glycol) dimethacrylates (PEGDM), poly(ethylène glycol) urethane-dimethacrylates (PEGUDM).

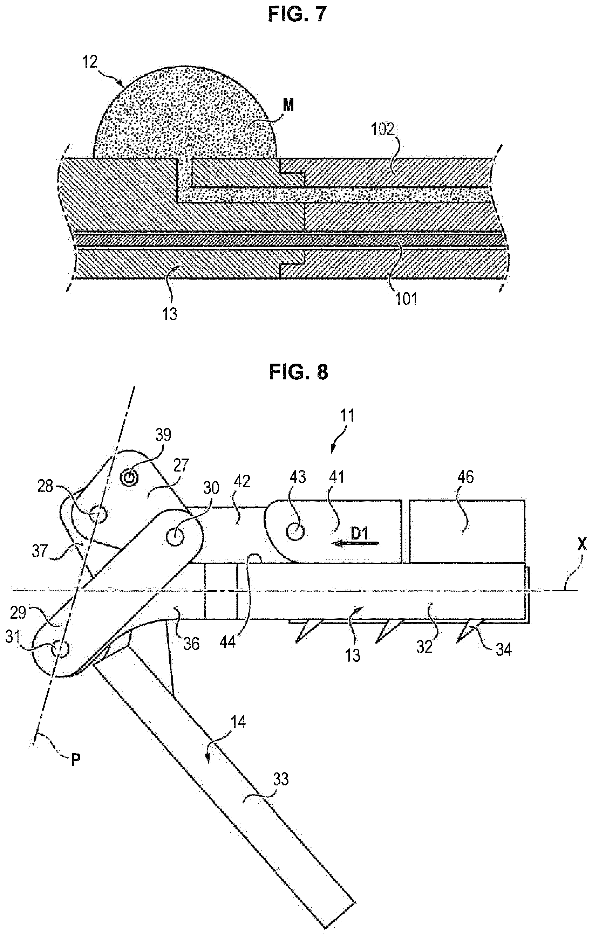

La figure 7 représente de manière schématique une étape de gonflage du ballonnet. Le matériau de remplissage M est injecté à l'intérieur de la cavité interne du ballonnet 12 via un canal d'injection s'étendant à l'intérieur du cathéter de largage 102 et du corps 13 de la mâchoire 11 auquel est fixé le ballonnet 12.Le ballonnet 12 est de préférence formé d'une membrane en matériau élastique, étanche, biocompatible et résistant à l'usure. Le matériau de la membrane est par exemple choisi parmi les polyamides (notamment le Nylon^{®}), les polytéréphtalates d'éthylène (« PET »), ou encore les silicones.

L'utilisation d'un ballonnet gonflable permet d'adapter les dimensions du ballonnet à l'importance de la fuite mitrale ainsi qu'à la morphologie du patient. L'opérateur peut en effet ajuster le volume du ballonnet jusqu'à disparition de la fuite mitrale. Le gonflage du ballonnet peut être contrôlé en temps réel à l'échographie trans-œsophagienne (ETO).

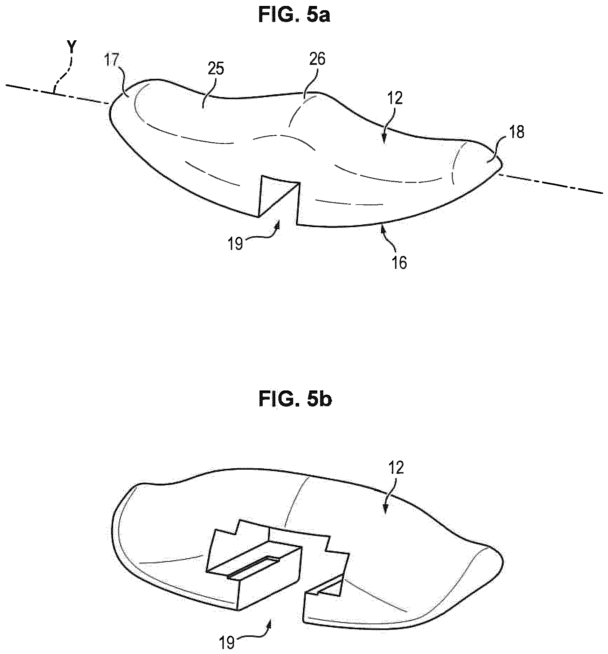

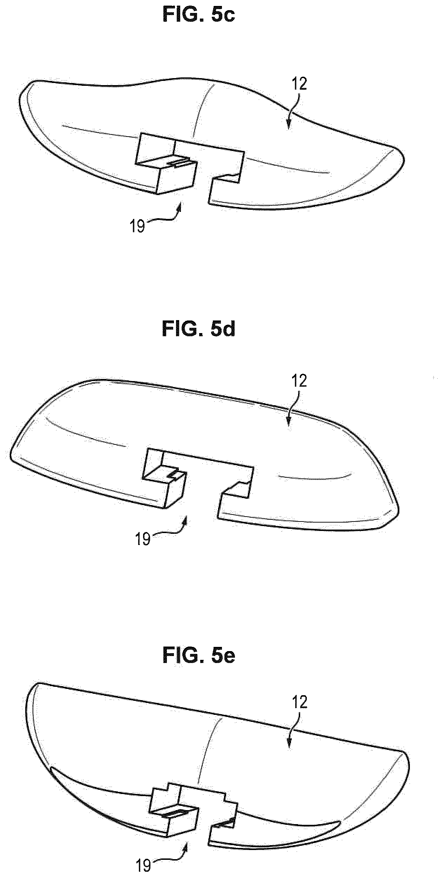

Un premier exemple de ballonnet 12 est illustré à la figure 5a.

Le ballonnet 12 présente une forme globalement allongée s'étendant suivant un axe transversal Y parallèle au premier axe de rotation 15. Ainsi, le ballonnet 12 s'étend le long des feuillets antérieur et postérieur FA et FP de la valve mitrale VM, lorsque l'implant 10 est fixé au feuillet postérieur FP, et épouse au mieux la portion O1 d'ouverture subsistant entre les feuillets antérieur et postérieur FA et FP lors de la systole, de manière à obturer la portion O1 d'ouverture subsistante.

Plus précisément, le ballonnet 12 présente globalement une forme arquée (ou une forme de croissant). Le ballonnet 12 présente une première face 16 agencée en regard de la mâchoire 11, et une deuxième face 25, opposée à la première face 16. La première face 16 est par exemple convexe entre chacune des extrémités 17, 18 du ballonnet 12 suivant l'axe transversal Y. La première face 16 du ballonnet 12 est propre à épouser la courbure du feuillet postérieur. En effet, la ligne de coaptation « naturelle » entre les feuillets antérieur et postérieur de la valve mitrale a une forme concave vers la chambre de chasse (canal aortique) ventriculaire gauche. La forme convexe du ballonnet 12 respecte ainsi la morphologie de la valve.

Une rainure 19 peut en outre être ménagée dans la première face 16 du ballonnet 12 suivant l'axe longitudinal X pour accueillir la mâchoire 11 et notamment le corps 13. De préférence, la rainure 19 traverse de part en part le ballonnet 12.

La deuxième face 25 du ballonnet 12 peut présenter une surépaisseur 26 globalement ménagée à mi-distance entre les extrémités transversales 17, 18 du ballonnet 12. Ainsi, le ballonnet 12 épouse au mieux la forme de la portion O1 d'ouverture subsistant entre les feuillets antérieur et postérieur FA et FP lors de la systole. L'obturation de la portion O1 d'ouverture est de cette manière plus efficace.

D'autres exemples de ballonnets sont illustrés sur les figures 5b à 5e.

Comme illustré sur la figure 4, l'implant 10 comprend en outre une languette de support 20 par l'intermédiaire de laquelle le ballonnet 12 est fixé à la mâchoire 11. Selon un mode de réalisation possible, le ballonnet 12 peut être fixé sur la languette de support 20, par exemple par couture, par soudure, par collage ou après trempage, et la languette de support 20 est à son tour fixée sur la mâchoire 11.

Sur la figure 4, la languette de support 20 s'étend suivant l'axe transversal Y. La languette de support 20 présente une première face 21 fixée à la première face 16 du ballonnet 12 et une deuxième face 22, opposée à la première face 21.

La languette de support 20 est formée en un matériau à mémoire de forme, présentant par exemple la forme d'une grille. Un matériau à mémoire de forme est un matériau qui a la capacité de garder en mémoire une forme initiale et de retourner à cette forme initiale après déformation. Ainsi, un matériau à mémoire de forme présente par exemple une première forme sur une première plage de température inférieure à une température critique Tc et une deuxième forme sur une deuxième plage de température supérieure à la température critique Tc. Les variations de température autour de la température critique Tc permettent ainsi de faire varier la forme du matériau. Le matériau à mémoire de forme est choisi de manière à présenter une température critique comprise entre 20°C et 37°C.

Dans un premier état de forme, la languette de support 20 est par exemple repliée sur elle-même. Dans ce premier état de forme, le ballonnet 12 est dégonflé. La languette de support 20 permet ainsi de limiter le volume de l'implant 10, lorsque ce dernier est acheminé vers le cœur du patient, à l'intérieur du cathéter principal 100. La languette de support 20 se trouve dans le premier état de forme tant que sa température est située dans une première plage de température inférieure à la température critique Tc du matériau. La languette de support 20 se trouve dans le premier état de forme lorsque la languette de support 20 est à une température inférieure à la température critique, notamment lorsqu'elle se trouve plongée dans un milieu ambiant, tel que l'air ambiant, ayant une température d'environ 20°C.

Dans un deuxième état de forme, la languette de support 20 est déployée. A l'état déployé, la première face 21 de la languette de support 20 est concave entre chacune de ses extrémités 23, 24 suivant l'axe transversal Y. La languette de support 20 donne ainsi à la première face 16 du ballonnet 12 sa forme convexe. Dans ce deuxième état de forme, le ballonnet 12 peut être gonflé pour obturer la portion O1 d'ouverture de la valve mitrale VM restée ouverte lors de la systole. La languette de support 20 se trouve dans le deuxième état de forme lorsque la température de la languette de support 20 est supérieure à la température critique, notamment lorsqu'elle se trouve plongée dans un milieu ambiant, tel que le sang, ayant une température de 37°C.

La température de la languette de support 20 varie automatiquement lorsque la languette de support 20 entre en contact avec le sang. Lorsque la température du milieu dans lequel se trouve plongée la languette de support 20 change, la languette de support 20 change de forme et passe du premier état de forme (replié) au deuxième état de forme (déployé).

De préférence, la languette de support 20 est formée en Nitinol. Le Nitinol est un alliage de nickel et de titane. Une fois au contact du sang à 37°C, la languette de support 20 passe de l'état replié à l'état déployé. La réalisation de la languette de support 20 en Nitinol est particulièrement avantageuse. En effet, le Nitinol est biocompatible et hémocompatible. Par ailleurs, il présente une très bonne résistance à la corrosion et est hyper-élastique.

Les figures 5b à 5e illustrent de manière schématique d'autres exemples de ballonnets pouvant faire partie de l'implant illustré sur la figure 4.

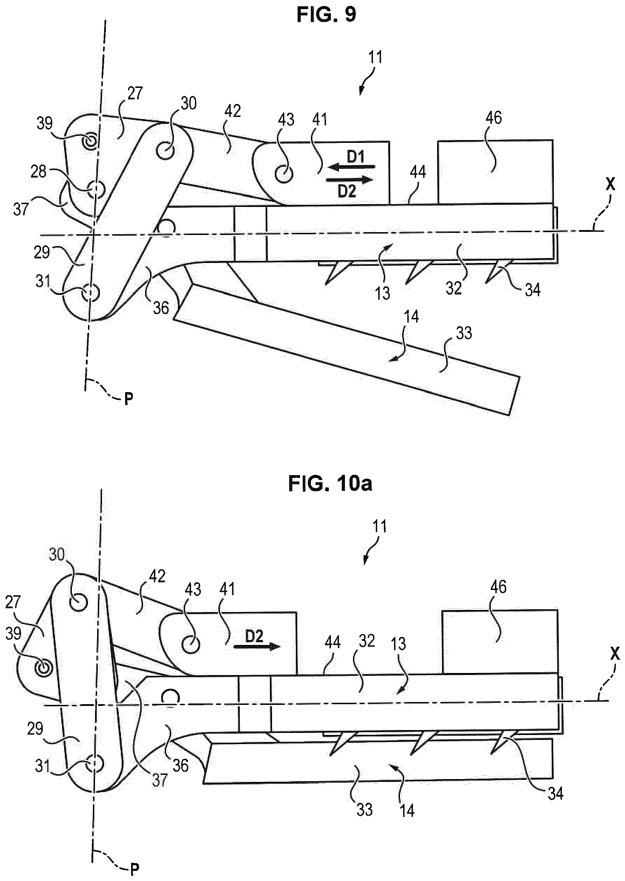

Les figures 7 et 10a-b montrent respectivement la mâchoire 11 dans la position ouverte et dans la position fermée. La figure 8 montre la mâchoire 11 dans une position intermédiaire entre les positions ouverte et fermée.

La mâchoire 11 comprend un dispositif d'entraînement en rotation 27 configuré pour entraîner en rotation le mors 14 par rapport au corps 13 autour du premier axe de rotation 15 entre la position ouverte et la position fermée.

Le dispositif d'entraînement en rotation 27 comprend par exemple une bielle articulée en rotation avec le mors 14 autour d'un deuxième axe de rotation 28. Le deuxième axe de rotation 28 est parallèle et non confondu avec le premier axe de rotation 15. Le déplacement de la bielle 27 entraîne ainsi le pivotement du mors 14 par rapport au corps 13 autour du premier axe de rotation 15 entre la position ouverte et la position fermée.

La mâchoire 11 peut également comprendre un dispositif de verrouillage 27, 29 configuré pour verrouiller la mâchoire 11 en position fermée. Le verrouillage de la mâchoire 11 en position fermée permet de diminuer les risques d'ouverture intempestive de la mâchoire 11 une fois l'implant 10 fixé au feuillet postérieur FP de la valve mitrale VM.

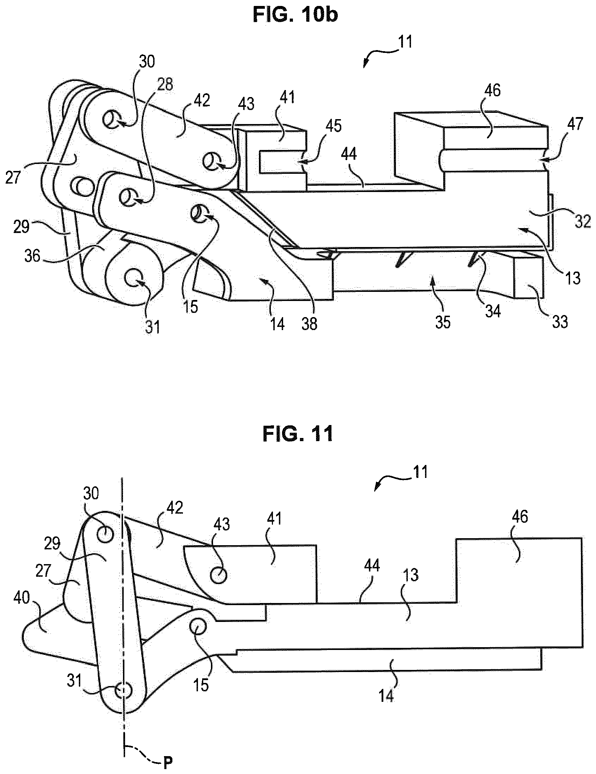

Les figures 10a et 10b montrent la mâchoire 11 dans une position fermée et verrouillée.

La mâchoire 11 comprend par exemple au moins un élément de bascule 29 articulé en rotation avec la bielle 27 autour d'un troisième axe de rotation 30 d'une part et avec le corps 13 de la mâchoire 11 autour d'un quatrième axe de rotation 31 d'autre part. Les troisième et quatrième axes de rotation 30, 31 sont parallèles et non confondus entre eux et avec les premier et deuxième axes de rotation 15, 28.

L'élément de bascule 29 et la bielle 27 forment ensemble une genouillère. La genouillère est configurée pour se déplacer entre une configuration déverrouillée dans laquelle le troisième axe de rotation 30 est situé d'un premier côté du plan P formé par les deuxième et quatrième axes de rotation 28, 31, la mâchoire 11 étant alors en position ouverte, et une configuration verrouillée dans laquelle le troisième axe de rotation 30 est situé d'un deuxième côté dudit plan P ,la mâchoire 11 étant alors en position fermée.

La mâchoire 11 comprend par exemple deux éléments de bascule 29 disposés de part et d'autre du corps 13 et de la bielle 27.

Le corps 13 et le mors 14 comprennent chacun une partie de prise 32, 33. Les parties de prise 32, 33 du corps 13 et du mors 14 sont agencées en regard l'une de l'autre et sont configurées pour enserrer le feuillet postérieur FP de la valve mitrale VM, lorsque la mâchoire 11 est en position fermée.

La partie de prise 32 du corps 13 accueille le ballonnet 12. Pour cela, la partie de prise 32 du corps 13 est par exemple insérée dans la rainure 19 ménagée à cet effet dans la première face du ballonnet 12.

La partie de prise 32 du corps 13 peut en outre présenter une pluralité de pointes 34 configurées pour se planter dans le feuillet postérieur FP de la valve mitrale VM, lorsque la mâchoire 11 enserre le feuillet postérieur FP de la valve mitrale. Les pointes 34 améliorent ainsi la prise de la mâchoire 11 sur le feuillet postérieur FP de la valve mitrale VM.

Les pointes 34 s'étendent par exemple globalement perpendiculairement depuis une face de ladite partie de prise 32 du corps 13 en regard du mors 14. Pour cela, une plaque munie des pointes 34 est par exemple fixée sur la face de la partie de prise 32 du corps 13 en regard du mors 14.

De préférence, une ou plusieurs ouvertures traversantes 35 sont ménagées dans la partie de prise 33 du mors 14, en regard des pointes 34 du corps 13. La ou les ouvertures 35 sont configurées pour accueillir les pointes 34 du corps 13, lorsque la mâchoire 11 est en position fermée. Ainsi, on s'assure que la fermeture de la mâchoire 11 n'est pas gênée par les pointes 34 du corps 13. La ou les ouvertures 35 peuvent être remplacées par un ou plusieurs logements.

Alternativement, les pointes 34 sont supportées par la partie de prise 33 du mors 14, par exemple par l'intermédiaire de la plaque, et le cas échéant la ou les ouvertures 35 sont ménagées dans la partie de prise 32 du corps 13.

Le corps 13 et le mors 14 comprennent chacun une partie d'actionnement 36, 37 accueillant respectivement les premier et quatrième axes de rotation 15, 31 et les premier et deuxième axes de rotation 15, 28. Les parties d'actionnement 36, 37 du corps 13 et du mors 14 sont agencées en regard l'une de l'autre.

Le premier axe de rotation 15 est par exemple situé sur la partie d'actionnement 36 du corps 13 entre la partie de prise 32 dudit corps 13 et le quatrième axe de rotation 31.

Le premier axe de rotation 15 est par exemple situé sur la partie d'actionnement 37 du mors 14 entre la partie de prise 33 dudit mors 14 et le deuxième axe de rotation 28.

Le deuxième axe de rotation 28 est par exemple positionné d'un côté opposé de la partie de prise 33 du mors 14, tandis que le quatrième axe de rotation 31 est positionné d'un côté opposé de la partie de prise 32 du corps 13.

Pour cela, une ouverture 38 traversante est par exemple ménagée dans la partie d'actionnement 36 du corps 13 pour accueillir la partie d'actionnement 37 du mors 14 et permettre au deuxième axe de rotation 28 de se situer d'un côté opposé à la partie de prise 33 du mors 14. Le corps 13 et le mors 14 se croisent ainsi au niveau du premier axe de rotation 15.

La mâchoire 11 peut en outre comprendre un dispositif de blocage 39, 40 configuré pour bloquer la genouillère 27, 29 en configuration verrouillée. Le blocage de la mâchoire 11 en position fermée et verrouillée permet de diminuer davantage les risques d'ouverture intempestive de la mâchoire 11 une fois l'implant 10 fixé au feuillet postérieur FP de la valve mitrale VM.

Pour cela, la bielle 27 comprend par exemple une butée 39 configurée pour venir en contact avec le ou les éléments de bascule 29 en configuration verrouillée de la genouillère. La butée 39 est par exemple formée par un axe parallèle aux deuxième et troisième axes de rotation 18, 20. Un exemple de mâchoire 11 avec la butée 39 est illustré aux figures 7 à 9.

En variante, le mors 14 comprend une protubérance 40 s'étendant depuis la partie d'actionnement 37 du mors 14 et configurée pour venir en contact avec la bielle 27 en configuration verrouillée de la genouillère. Un exemple de mâchoire 11 avec la protubérance 40 est illustré à la figure 11.

La mâchoire 11 peut en outre comprendre un dispositif de déplacement 41, 42 configuré pour déplacer la bielle 27 et ainsi entraîner le pivotement du mors 14 par rapport au corps 13 autour du premier axe de rotation 15 entre la position ouverte et la position fermée.

Le dispositif de déplacement 41, 42 comprend par exemple un élément poussoir 41 configuré pour se déplacer en translation suivant l'axe longitudinal X par rapport au corps 13 de la mâchoire 11, et un élément de levier 42 articulé en rotation par rapport à la bielle 27 autour du troisième axe de rotation 30 d'une part et par rapport à l'élément poussoir 41 autour d'un cinquième axe de rotation 43. Le cinquième axe de rotation 43 est parallèle et non confondu avec les premier, deuxième, troisième et quatrième axes de rotation 15, 28, 30, 31.

Ainsi, lorsque l'élément poussoir 41 se déplace en translation suivant l'axe longitudinal X par rapport au corps 13, il entraîne, par l'intermédiaire de l'élément de levier 42, le déplacement du troisième axe de rotation 30. Lorsque l'élément poussoir 41 est déplacé dans une première direction D1, le mors 14 pivote autour du premier axe de rotation 15 vers la position serrée PS et le troisième axe de rotation 30 est entrainé vers la configuration verrouillée. Lorsque l'élément poussoir 41 est déplacé dans une deuxième direction D2, opposée à la première direction D1, le mors 14 pivote autour du premier axe de rotation 15 vers la position écartée et le troisième axe de rotation 30 s'écarte de la configuration verrouillée.

De préférence, l'élément poussoir 41 est configuré pour se déplacer en translation suivant l'axe longitudinal X par glissement contre une face 44 du corps 13, opposée à la partie de prise 33 du mors 14. Ainsi, le déplacement de l'élément poussoir 41 est guidé par le corps 13.

L'élément poussoir 41 est par exemple pourvu d'une ouverture 45 s'étendant suivant l'axe longitudinal X. L'ouverture 45 est configurée pour coopérer avec l'extrémité distale de la tige 101. Ainsi, l'opérateur peut manipuler l'extrémité proximale de la tige 101 pour déplacer l'élément poussoir 41 et donc activer l'ouverture et la fermeture de la mâchoire 11, et le cas échéant son verrouillage. Un exemple d'ouverture 45 de l'élément poussoir 41 est illustré à la figure 10b.

Le corps 13 peut en outre comprendre un élément de guidage 46 configuré pour guider la translation de l'extrémité distale de la tige 101 suivant l'axe longitudinal X.

L'élément de guidage 46 s'étend par exemple depuis la face 44 du corps 13 opposée à la partie de prise 33 du mors 24 et comprend une ouverture 47 traversante s'étendant suivant l'axe longitudinal X et configurée pour accueillir de manière coulissante l'extrémité distale de la tige 101. L'ouverture 47 de l'élément de guidage 46 et l'ouverture 45 de l'élément poussoir 40 sont coaxiales et agencées en regard l'une de l'autre. L'ouverture 47 guide ainsi le déplacement en translation de l'extrémité distale de la tige 101 suivant l'axe longitudinal X qui peut alors entraîner l'élément poussoir 41 en translation suivant ledit axe longitudinal X et ainsi ouvrir et fermer la mâchoire 11. Un exemple d'ouverture 47 de l'élément de guidage 34 est illustré à la figure 10b.

La mâchoire 11 est par exemple recouverte d'un revêtement en Dacron^{®}. Ce matériau favorise l'endothélialisation (colonisation d'un tissu par les cellules endothéliales) de l'implant, ce qui améliore sa tenue sur le site d'implantation et diminue le risque de formation de thrombus (obturation d'un vaisseau sanguin). Autrement dit, la mâchoire 11 est par exemple formée en titane et une fine couche de revêtement en Dacron^{®} la recouvre. En variante, la mâchoire 11 est formée en un alliage de chrome et de cobalt.

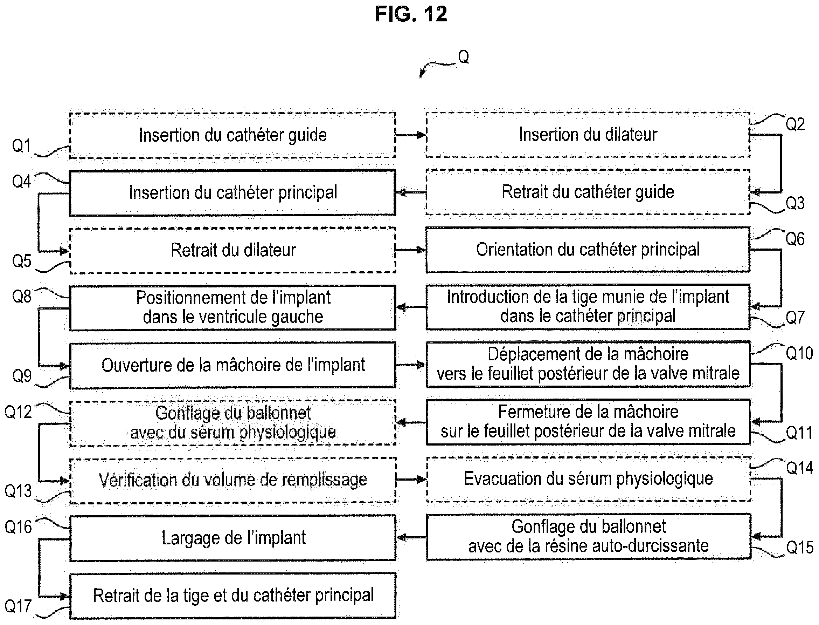

La figure 12 est un diagramme illustrant différentes étapes d'un procédé P de pose de l'implant 10 par voie trans-septale à titre d'exemple.

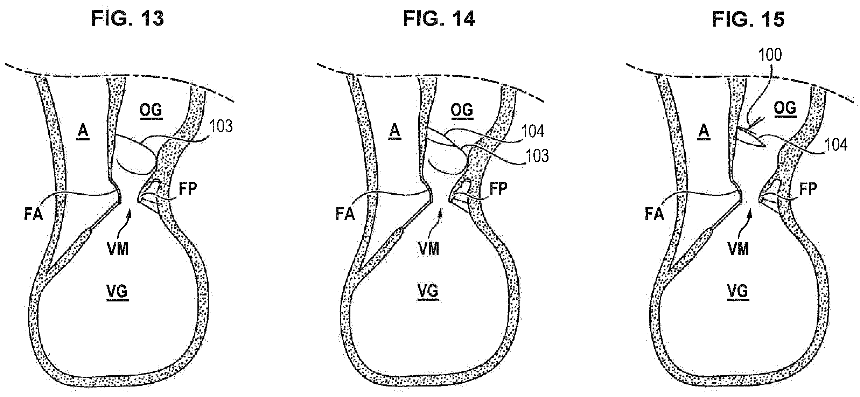

Au cours d'une première étape Q1, un guide 103 est inséré dans la veine fémorale, jusqu'à l'oreillette droite OD puis un cathétérisme trans-septal est réalisé (à travers le septum inter-atrial) jusqu'à l'oreillette gauche OG et l'anneau mitral AM. La première étape Q1 est illustrée à la figure 13.

Au cours d'une deuxième étape Q2, un dilatateur 104 est enfilé sur le guide 103,à son tour inséré jusqu'à l'oreillette gauche OG. Le dilatateur 104 est ainsi guidé par le guide 103 jusqu'à l'oreillette gauche OG. Le dilatateur 104 a pour effet de dilater les tissus du septum inter-atrial pour permettre l'insertion du cathéter principal 100. La deuxième étape Q2 est illustrée à la figure 14.

Au cours d'une troisième étape Q3, le cathéter guide 103 est retiré.

Au cours d'une quatrième étape Q4, le cathéter principal 100 est à son tour inséré sur le guide, à travers le septum inter-atrial jusqu'à l'oreillette gauche OG. Pour cela, le cathéter principal 100 est par exemple enfilé autour du dilatateur 104. La quatrième étape Q4 est illustrée à la figure 15.

Au cours d'une cinquième étape Q5, le dilatateur 104 est retiré.

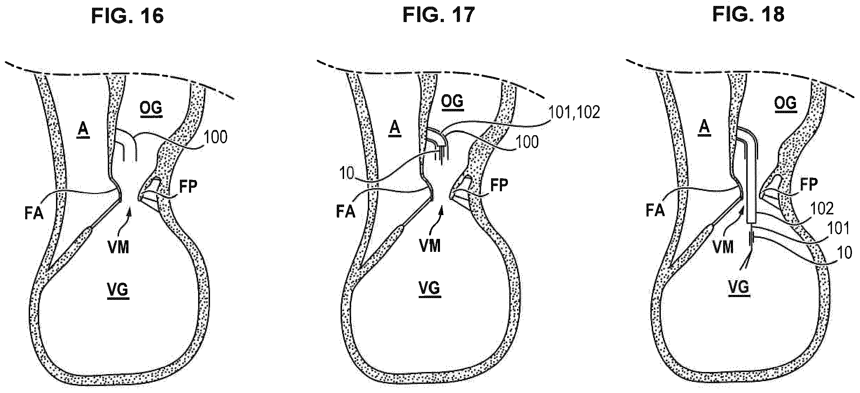

Au cours d'une sixième étape Q6, l'extrémité distale 105 du cathéter principal 100 est orientée de sorte à s'étendre de manière globalement coaxiale avec l'anneau mitral AM. L'extrémité distale 105 du cathéter principal 100 comprend ainsi une partie orientable. La sixième étape Q6 est illustrée à la figure 16.

Au cours d'une septième étape Q7, la tige 101 dont l'extrémité distale coopère avec l'implant 10, et le cas échéant le cathéter de largage 102, sont introduits dans le cathéter principal 100. La tige 101 et le cathéter de largage 102 sont alors dans la première configuration dans laquelle la tige 101 et le cathéter de largage 102 se déplacent d'un seul tenant. La mâchoire 11 est en position fermée. Le ballonnet 12 à l'état dégonflé et le cas échéant la languette de support 20 est dans le premier état de forme. La septième étape Q7 est illustrée à la figure 17.

Au cours d'une huitième étape Q8, l'extrémité distale de la tige 101 et le cas échéant celle du cathéter de largage 102 sortent du cathéter principal 100 et traversent la valve mitrale VM jusqu'à ce que l'extrémité distale du cathéter de largage 102 soit positionnée dans le ventricule gauche VG. Le ballonnet 12 de l'implant 10 est alors orienté vers le feuillet antérieur FA de la valve mitrale VM, tandis que le mors 14 de la mâchoire 11 est orienté vers le feuillet postérieur FP de la valve mitrale VM. La mâchoire 11 est toujours en position fermée. Le ballonnet 12 est à l'état dégonflé et le cas échéant, au contact du sang, la languette de support 20 passe du premier état de forme (replié) au deuxième état de forme (déployé). La huitième étape Q8 est illustrée à la figure 18.

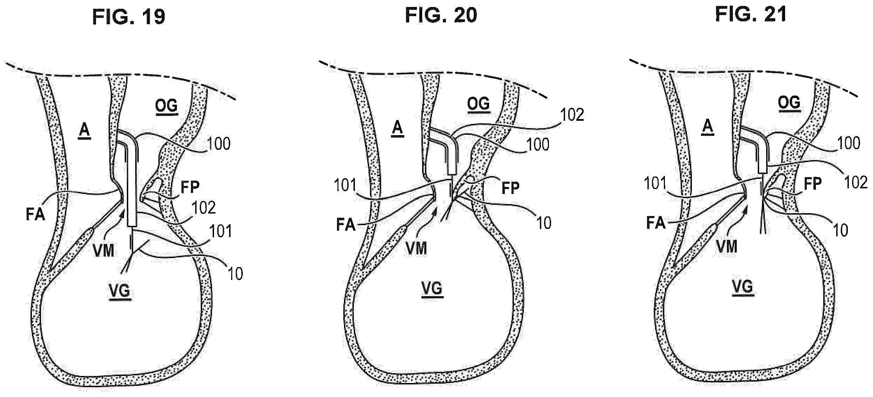

Au cours d'une neuvième étape Q9, la mâchoire 11 est ouverte. Pour cela, la tige 101 tire par exemple l'élément poussoir 41 vers l'extrémité du corps 13 portant le ballonnet 12. La tige 101 et le guide de tige 102 sont alors dans la deuxième configuration dans laquelle la tige 101 est libre de coulisser à l'intérieur du guide de tige 102. La neuvième étape Q9 est illustrée à la figure 19.

Au cours d'une dixième étape Q10, la tige 101 et le cas échéant le cathéter de largage 102 se déplacent vers la valve mitrale VM de sorte que le feuillet postérieur FP de la valve mitrale VM soit disposé entre le corps 13 et le mors 14 de la mâchoire 11 toujours ouverte. La tige 101 et le cathéter de largage 102 sont alors dans la première configuration dans laquelle la tige 101 et le cathéter de largage 102 se déplacent d'un seul tenant. La dixième étape Q10 est illustrée à la figure 20.

Au cours d'une onzième étape Q11, la mâchoire 11 est fermée et le cas échéant verrouillée. L'implant 10 est ainsi fixé au feuillet postérieur FP de la valve mitrale VM. Pour cela, la tige 101 pousse par exemple l'élément poussoir 41 vers l'aval en direction de l'apex du corps 13 de l'implant 10 opposée au ballonnet 12. La tige 101 et le cathéter de largage 102 sont alors dans la deuxième configuration dans laquelle la tige 101 est libre de coulisser à l'intérieur du cathéter de largage 102. La onzième étape Q11 est illustrée à la figure 21.

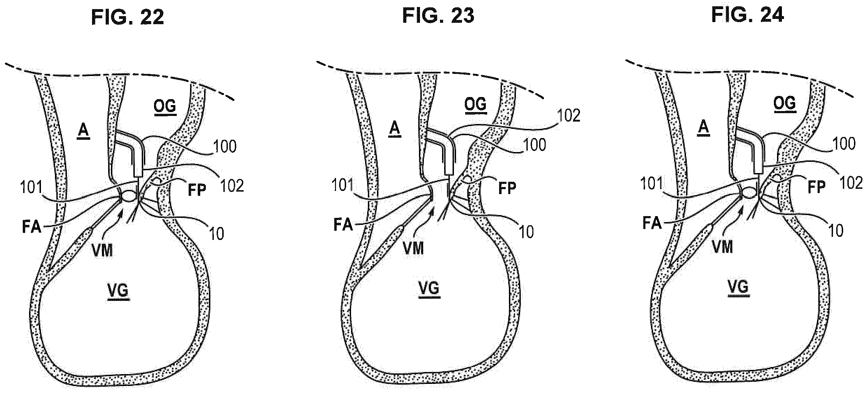

Au cours d'une douzième étape Q12, le ballonnet 12 est gonflé avec du sérum physiologique, et le volume de sérum physiologique remplissant le ballonnet 12 est déterminé. La douzième étape Q12 est illustrée à la figure 22.

Au cours d'une treizième étape Q13, l'effet du gonflement du ballonnet 12 au volume de remplissage déterminé est vérifié par injection de sérum physiologique dans la cavité du ballonnet 12. Pour cela, l'opérateur regarde si le reflux de sang est suffisamment diminué voire supprimé grâce à une échocardiographie trans-œsophagienne (ETO). Si le volume de remplissage déterminé est jugé satisfaisant, le volume de remplissage déterminé est validé. Sinon, le volume de remplissage est ajusté puis vérifié à nouveau.

Au cours d'une quatorzième étape Q14, optionnelle, le sérum physiologique est retiré du ballonnet 12. La quatorzième étape Q14 est illustrée à la figure 23.

Au cours d'une quinzième étape Q15, le ballonnet 12 est gonflé avec un matériau de remplissage. Le volume de matériau de remplissage injecté dans le ballonnet 12 correspond par exemple au volume de remplissage déterminé à la douzième étape Q12. La quinzième étape Q15 est illustrée à la figure 24.

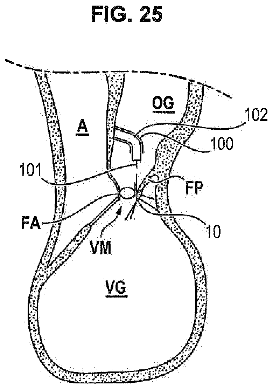

Au cours d'une seizième étape Q16, l'implant 10 est largué. Pour cela, la tige 101 est par exemple découplée de l'élément poussoir 41. La seizième étape Q16 intervient de préférence une fois la résine durcie. La seizième étape Q16 est illustrée à la figure 25.

Au cours d'une dix-septième étape Q17, la tige 101, le cas échéant le cathéter de largage 102 et le cathéter principal 100 sont retirés du corps du patient.

Toutes ces étapes peuvent être réalisées sous contrôle fluoroscopique cardiaque et sous contrôle ETO (échocardiographie trans-œsophagienne).

Le procédé Q de pose de l'implant 10 par voie percutanée est particulièrement avantageux car il présente des risques réduits pour le patient comparativement à une opération à cœur ouvert.

L'implant et le procédé tels que décrits précédemment ne sont pas uniquement destinés à une pose sur une valve mitrale. L'implant proposé peut également être posé sur d'autres valves cardiaques que la valve mitrale, en particulier l'autre valve auriculo-ventriculaire, c'est-à-dire la valve tricuspide.

## Revendications

1. Implant (10) pour une valve cardiaque comprenant deux feuillets, notamment une valve mitrale (VM), l'implant (10) comprenant :
- une mâchoire (11) comprenant un corps (13) et un mors (14) articulé en rotation avec le corps (13) pour pivoter entre une position ouverte et une position fermée dans laquelle le corps (13) et le mors (14) sont aptes à enserrer un premier feuillet parmi les feuillets de la valve cardiaque, de sorte à fixer l'implant (10) sur le premier feuillet,
- un dispositif de déplacement (41, 42) comprenant un élément poussoir (41), le dispositif de déplacement étant configuré pour déplacer le mors (14) par rapport au corps (13) depuis l'une parmi les positions ouverte et fermée à l'autre position, lorsque l'élément poussoir (41) est poussé par une tige (101),
caractérisé en ce l'implant comprend un ballonnet (12) fixé à la mâchoire (11) et configuré pour obturer au moins en partie une portion (O1) d'ouverture subsistant entre les feuillets de la valve cardiaque (VM) lors d'une systole, lorsque l'implant (10) est fixé sur le premier feuillet, de sorte à limiter un reflux de sang à travers ladite portion (O1) d'ouverture subsistant lors de la systole.

2. Implant (10) selon la revendication 1, configuré pour être inséré dans un cathéter principal 100, de sorte à être implanté par voie percutanée ou par voie trans-apicale.

3. Implant (10) selon la revendication 1 ou la revendication 2, dans lequel le ballonnet (12) est gonflable.

4. Implant (10) selon l'une des revendications 1 à 3, dans lequel le ballonnet (12) présente une forme globalement allongée s'étendant suivant un axe transversal (Y) parallèle au premier axe de rotation (15).

5. Implant (10) selon la revendication 4, dans lequel le ballonnet (12) présente une première face (16) agencée en regard de la mâchoire (11), la première face (16) étant convexe entre chacune des extrémités (17, 18) du ballonnet (12) suivant l'axe transversal (Y).

6. Implant (10) selon l'une des revendications 1 à 5, comprenant une languette de support (20) fixée au ballonnet (12), ladite languette de support (20) comprenant un matériau à mémoire de forme présentant dans une première gamme de températures, un premier état de forme dans lequel la languette de support (20) est repliée et, dans une deuxième gamme de températures, un deuxième état de forme dans lequel la languette de support (20) est déployée.

7. Implant (10) selon l'une des revendications précédentes, dans lequel la mâchoire (11) comprend un dispositif de verrouillage (27, 29) configuré pour verrouiller la mâchoire (11) en position fermée, et dans lequel le dispositif de déplacement (41, 42) est configuré pour agir sur le dispositif de verrouillage pour que le mors soit verrouillé en position fermée, lorsque le dispositif de déplacement déplace le mors (14) par rapport au corps (13) dans la position fermée.

8. Implant (10) selon l'une des revendications précédentes, dans lequel le dispositif de verrouillage (27, 29) comprend une genouillère, dans lequel le mors (14) est articulé en rotation autour d'un premier axe de rotation par rapport au corps (13), et dans lequel la mâchoire (11) comprend une bielle (27) articulée en rotation avec le mors (14) autour d'un deuxième axe de rotation (28), ledit deuxième axe de rotation (28) étant parallèle avec le premier axe de rotation (15), dans lequel la mâchoire (11) comprend au moins un élément de bascule (29) articulé en rotation avec la bielle (27) autour d'un troisième axe de rotation (30) d'une part et avec le corps (13) de la mâchoire (11) autour d'un quatrième axe de rotation (31) d'autre part, les troisième et quatrième axes de rotation (30, 31) étant parallèles entre eux et avec les premier et deuxième axes de rotation (15, 28), et dans lequel l'élément de bascule (29) et la bielle (27) forment ensemble la genouillère, dans lequel la genouillère est configurée pour se déplacer entre une configuration déverrouillée dans laquelle le troisième axe de rotation (30) est situé d'un premier côté du plan (P) formé par les deuxième et quatrième axes de rotation (28, 31) et la mâchoire (11) est en position ouverte, et une configuration verrouillée dans laquelle le troisième axe de rotation (30) est situé d'un deuxième côté dudit plan (P) et la mâchoire (11) est en position fermée, dans lequel la mâchoire (11) comprend un dispositif de blocage (39, 40) configuré pour bloquer la genouillère (27, 29) en configuration verrouillée, dans lequel le dispositif de blocage (39, 40) comprend
- une butée (39) de la bielle (27) configurée pour venir en contact avec le ou les éléments de bascule (29), lorsque la genouillère (27, 29) est en configuration verrouillée, ou
- une protubérance (40) du mors (14) configurée pour venir en contact avec la bielle (27), lorsque la genouillère (27, 29) est en configuration verrouillée.

9. Implant (10) selon l'une des revendications précédentes, dans lequel le corps s'étend le long d'un axe longitudinal (X), le mors (14) est articulé en rotation par rapport au corps (13) autour d'un premier axe de rotation perpendiculaire à l'axe longitudinal (X), et l'élément poussoir (41) est configuré pour se déplacer en translation suivant l'axe longitudinal (X) par rapport au corps (13) de la mâchoire (11), et dans lequel le dispositif de déplacement comprend par ailleurs un élément de levier (42) articulé en rotation par rapport à la bielle (27) autour du troisième axe de rotation (30) d'une part et par rapport à l'élément poussoir (41) autour d'un cinquième axe de rotation (43) parallèle avec le premier axe de rotation (15, 28, 30, 31) d'autre part, dans lequel le corps (13) et le mors (14) comprennent chacun une partie de prise (32, 33) agencées en regard l'une de l'autre et configurées pour enserrer le premier feuillet de la valve cardiaque, par exemple le feuillet postérieur (FP) d'une valve mitrale (VM), lorsque la mâchoire (11) est en position fermée, et dans lequel l'élément poussoir (41) est en outre configuré pour se déplacer en translation suivant l'axe longitudinal (X) par glissement contre une face (44) du corps (13), opposée à la partie de prise (33) du mors (14).

10. Implant (10) selon l'une des revendications précédentes, dans lequel le corps s'étend le long d'un axe longitudinal (X), et dans lequel l'élément poussoir (41) est pourvu d'une ouverture (45) s'étendant suivant l'axe longitudinal (X) et configurée pour coopérer avec l'extrémité distale de la tige (101), dans lequel le corps (13) comprend un élément de guidage (46) configuré pour guider une translation de l'extrémité distale de la tige (101) suivant l'axe longitudinal (X).

11. Implant (10) selon la revendication 10, dans lequel l'élément de guidage (46) s'étend depuis la face (44) du corps (13) opposée à la partie de prise (33) du mors (14) et comprend une ouverture (47) coaxiale avec et en regard de l'ouverture (45) de l'élément poussoir (40), ladite ouverture (47) de l'élément de guidage (46) étant configurée pour accueillir de manière coulissante l'extrémité distale de la tige (101).

12. Implant (10) selon l'une des revendications précédentes, dans lequel le corps (13) et le mors (14) comprennent chacun une partie de prise (32, 33) agencées en regard l'une de l'autre et configurées pour enserrer le premier feuillet de la valve cardiaque, par exemple le feuillet postérieur (FP) d'une valve mitrale (VM), lorsque la mâchoire (11) est en position fermée, et dans lequel la partie de prise (32, 33) du corps (13) ou du mors (14) comprend une pluralité de pointes (34) configurées pour se planter dans le premier feuillet, lorsque la mâchoire (11) enserre le premier feuillet.

13. Implant (10) selon la revendication précédente, dans lequel au moins une ouverture (35) est ménagée dans la partie de prise (33, 32) du mors (14) ou du corps (13), en regard des pointes (34) du corps (13).

14. Ensemble comprenant :
- un implant (10) pour une valve cardiaque selon l'une des revendications précédentes,
- une tige (101) dont l'extrémité distale est configurée pour coopérer avec l'élément poussoir de l'implant (10), de sorte à ouvrir et à fermer ladite mâchoire (11).

15. Ensemble selon la revendication précédente, comprenant en outre un cathéter principal (100) configuré pour accueillir l'implant (10) et la tige (101).

## Patentansprüche

1. Implantat (10) für eine Herzklappe, umfassend zwei Segel, insbesondere eine Mitralklappe (VM), wobei das Implantat (10) umfasst:
- eine Spannbacke (11), umfassend einen Körper (13) und eine Backe (14), die mit dem Körper (13) rotatorisch gelenkig ist, um zwischen einer geöffneten Position und einer geschlossenen Position zu schwenken, in welcher der Körper (13) und die Backe (14) imstande sind, ein erstes Segel von den Segeln der Herzklappe einzuklemmen, so dass das Implantat (10) auf dem ersten Segel befestigt ist,
- eine Verlagerungsvorrichtung (41, 42), umfassend ein Schieberelement (41), wobei die Verlagerungsvorrichtung ausgelegt ist, um die Backe (14) im Verhältnis zum Körper (13) aus einer von der geöffneten und geschlossenen Position in die andere Position zu verlagern, wenn das Schieberelement (41) von einer Stange (101) geschoben wird,
**dadurch gekennzeichnet, dass** das Implantat einen Ballon (12) umfasst, der an der Spannbacke (11) befestigt und ausgelegt ist, um einen zwischen den Segeln der Herzklappe (VM) bei einer Systole verbleibenden Öffnungsabschnitt (O1) mindestens teilweise zu verschließen, wenn das Implantat (10) auf dem ersten Segel befestigt ist, so dass ein Blutrückfluss durch den bei der Systole verbleibenden Öffnungsabschnitt (O1) begrenzt wird.

2. Implantat (10) nach Anspruch 1, das ausgelegt ist, um in einen Hauptkatheter (100) derart eingesetzt zu sein, dass es auf perkutanem Weg oder auf transapikalem Weg implantiert wird.

3. Implantat (10) nach Anspruch 1 oder Anspruch 2, wobei der Ballon (12) aufblasbar ist.

4. Implantat (10) nach einem der Ansprüche 1 bis 3, wobei der Ballon (12) eine allgemein längliche Form aufweist, die sich gemäß einer transversalen Achse (Y) erstreckt, die parallel zur ersten Rotationsachse (15) ist.

5. Implantat (10) nach Anspruch 4, wobei der Ballon (12) eine erste Fläche (16) aufweist, die der Spannbacke (11) zugewandt eingerichtet ist, wobei die erste Fläche (16) zwischen jedem der Enden (17, 18) des Ballons (12) gemäß der transversalen Achse (Y) konvex ist.

6. Implantat (10) nach einem der Ansprüche 1 bis 5, umfassend eine am Ballon (12) befestigte Stützlasche (20), wobei die Stützlasche (20) ein Material mit Formengedächtnis umfasst, das in einem ersten Temperaturbereich einen ersten Formzustand umfasst, in dem die Stützlasche (20) gefaltet ist, und in einem zweiten Temperaturbereich einen zweiten Formzustand, in dem die Stützlasche (20) entfaltet ist.

7. Implantat (10) nach einem der vorangehenden Ansprüche, wobei die Spannbacke (11) eine Verriegelungsvorrichtung (27, 29) umfasst, die ausgelegt ist, um die Spannbacke (11) in geschlossener Position zu verriegeln, und wobei die Verlagerungsvorrichtung (41, 42) ausgelegt ist, um auf die Verriegelungsvorrichtung einzuwirken, damit die Backe in geschlossener Position verriegelt ist, wenn die Verlagerungsvorrichtung die Backe (14) im Verhältnis zum Körper (13) in die geschlossene Position verlagert.

8. Implantat (10) nach einem der vorangehenden Ansprüche, wobei die Verriegelungsvorrichtung (27, 29) ein Kniegelenk umfasst, wobei die Backe (14) um eine erste Rotationsachse im Verhältnis zum Körper (13) rotatorisch gelenkig ist, und wobei die Spannbacke (11) eine Pleuelstange (27) umfasst, die mit der Backe (14) um eine zweite Rotationsachse (28) rotatorisch gelenkig ist, wobei die zweite Rotationsachse (28) mit der ersten Rotationsachse (15) parallel ist, wobei die Spannbacke (11) mindestens ein Wippelement (29) umfasst, das mit der Pleuelstange (27) um eine dritte Rotationsachse (30) zum einen und mit dem Körper (13) der Spannbacke (11) um eine vierte Rotationsachse (31) zum andren rotatorisch gelenkig ist, wobei die dritte und vierte Rotationsachse (30, 31) zueinander und mit der ersten und zweiten Rotationsachse (15, 28) parallel sind, und wobei das Wippelement (29) und die Pleuelstange (27) gemeinsam das Kniegelenk bilden, wobei das Kniegelenk ausgelegt ist, um sich zwischen einer entriegelten Konfiguration, in der sich die dritte Rotationsachse (30) auf einer ersten Seite der Ebene (P) befindet, die von der zweiten und vierten Rotationsachse (28, 31) gebildet wird, und die Spannbacke (11) in geöffneter Position ist, und einer verriegelten Konfiguration, in welcher sich die dritte Rotationsachse (30) auf einer zweiten Seite der Ebene (P) befindet und die Spannbacke (11) in geschlossener Position ist, zu verlagern, wobei die Spannbacke (11) eine Arretiervorrichtung (39, 40) umfasst, die ausgelegt ist, um das Kniegelenk (27, 29) in verriegelter Konfiguration zu arretieren, wobei die Arretiervorrichtung (39, 40) umfasst
- einen Anschlag (39) der Pleuelstange (27), der ausgelegt ist, um mit dem oder den Wippelementen (29) in Kontakt zu kommen, wenn das Kniegelenk (27, 29) in verriegelter Konfiguration ist, oder
- einen Vorsprung (40) der Backe (14), der ausgelegt ist, um mit der Pleuelstange (27) in Kontakt zu kommen, wenn das Kniegelenk (27, 29) in verriegelter Konfiguration ist.

9. Implantat (10) nach einem der vorangehenden Ansprüche, wobei sich der Körper entlang einer Längsachse (X) erstreckt, die Backe (14) im Verhältnis zum Körper (13) um eine erste Rotationsachse, die senkrecht zur Längsachse (X) ist, rotatorisch gelenkig ist, und das Schieberelement (41) ausgelegt ist, um sich translatorisch gemäß der Längsachse (X) im Verhältnis zum Körper (13) der Spannbacke (11) zu verlagern, und wobei die Verlagerungsvorrichtung im Übrigen ein Hebelelement (42) umfasst, das im Verhältnis zur Pleuelstange (27) um die dritte Rotationsachse (30) zum einen und im Verhältnis zum Schieberelement (41) um eine fünfte Rotationsachse (43), die mit der ersten Rotationsachse (15, 28, 30, 31) zum anderen parallel ist, rotatorisch gelenkig ist, wobei der Körper (13) und die Backe (14) jeweils einen Eingriffsteil (32, 33) umfassen, die einander zugewandt eingerichtet und ausgelegt sind, um das erste Segel der Herzklappe, beispielsweise das posteriore Segel (FP) einer Mitralklappe (VM), einzuklemmen, wenn die Spannbacke (11) in geschlossener Position ist, und wobei das Schieberelement (41) ferner ausgelegt ist, um sich translatorisch gemäß der Längsachse (X) durch Gleiten an einer Fläche (44) des Körpers (13) gegenüber dem Eingriffsteil (33) der Backe (14) zu verlagern.

10. Implantat (10) nach einem der vorangehenden Ansprüche, wobei sich der Körper entlang einer Längsachse (X) erstreckt und wobei das Schieberelement (41) mit einer Öffnung (45) versehen ist, die sich gemäß der Längsachse (X) erstreckt und ausgelegt ist, um mit dem distalen Ende der Stange (101) zusammenzuwirken, wobei der Körper (13) ein Führungselement (46) umfasst, das ausgelegt ist, um eine Translation des distalen Endes der Stange (101) gemäß der Längsachse (X) zu führen.

11. Implantat (10) nach Anspruch 10, wobei sich das Führungselement (46) ab der Fläche (44) des Körpers (13) gegenüber dem Eingriffsteil (33) der Backe (14) erstreckt und eine koaxiale Öffnung (47) mit und der Öffnung (45) des Schieberelements (40) zugewandt umfasst, wobei die Öffnung (47) des Führungselements (46) ausgelegt ist, um das distale Ende der Stange (101) gleitend zu empfangen.

12. Implantat (10) nach einem der vorangehenden Ansprüche, wobei der Körper (13) und die Backe (14) jeweils einen Eingriffsteil (32, 33) umfasst, die einander zugewandt eingerichtet und ausgelegt sind, um das erste Segel der Herzklappe, beispielsweise das posteriore Segel (FP) einer Mitralklappe (VM), einzuklemmen, wenn die Spannbacke (11) in geschlossener Position ist, und wobei der Eingriffsteil (32, 33) des Körpers (13) oder der Backe (14) eine Vielzahl von Spitzen (34) umfasst, die ausgelegt sind, um in das erste Segel einzudringen, wenn die Spannbacke (11) das erste Segel einklemmt.

13. Implantat (10) nach vorangehendem Anspruch, wobei mindestens eine Öffnung (35) im Eingriffsteil (33, 32) der Backe (14) oder des Körpers (13) den Spitzen (34) des Körpers (13) zugewandt ausgebildet ist.

14. Einheit, umfassend:
- ein Implantat (10) für eine Herzklappe nach einem der vorangehenden Ansprüche,
- eine Stange (101), deren distales Ende ausgelegt ist, um mit dem Schieberelement des Implantats (10) derart zusammenzuwirken, dass die Spannbacke (11) geöffnet und geschlossen wird.

15. Einheit nach vorangehendem Anspruch, umfassend ferner einen Hauptkatheter (100), der ausgelegt ist, um das Implantat (10) und die Stange (101) aufzunehmen.

## Claims

1. An implant (10) for a heart valve comprising two leaflets, in particular a mitral valve (VM), the implant (10) comprising:
- a jaw (11) comprising a body (13) and a clamp (14) hinged in rotation with the body (13) to pivot between an open position and a closed position in which the body (13) and the clamp (14) are capable of enclosing a first leaflet among the leaflets of the heart valve, so as to fasten the implant (10) on the first leaflet,
- a displacement device (41, 42) comprising a pusher element (41), the displacement device being configured to displace the clamp (14) relative to the body (13) from one of the open and closed positions to the other position, when the pusher element (41) is pushed by a rod (101),
**characterised in that** the implant comprises a balloon (12) fastened to the jaw (11) and configured to at least partially seal a portion (01) of opening remaining between the leaflets of the heart valve (VM) during a systole, when the implant (10) is fastened on the first leaflet, so as to limit a backflow of blood through said portion (01) of opening remaining during the systole.

2. The implant (10) according to claim 1, configured to be inserted into a main catheter 100, so as to be implanted percutaneously or transapically.

3. The implant (10) according to claim 1 or claim 2, wherein the balloon (12) is inflatable.

4. The implant (10) according to one of claims 1 to 3, wherein the balloon (12) has a generally elongated shape extending along a transverse axis (Y) parallel to the first axis of rotation (15).

5. The implant (10) according to claim 4, wherein the balloon (12) has a first face (16) arranged opposite to the jaw (11), the first face (16) being convex between each of the ends (17, 18) of the balloon (12) along the transverse axis (Y).

6. The implant (10) according to one of claims 1 to 5, comprising a support tab (20) fastened to the balloon (12), said support tab (20) comprising a shape memory material having in a first temperature range, a first shape state in which the support tab (20) is folded back and, in a second temperature range, a second shape state in which the support tab (20) is deployed.

7. The implant (10) according to one of the preceding claims, wherein the jaw (11) comprises a locking device (27, 29) configured to lock the jaw (11) in the closed position, and wherein the displacement device (41, 42) is configured to act on the locking device so that the clamp is locked in the closed position, when the displacement device displaces the clamp (14) relative to the body (13) in the closed position.

8. The implant (10) according to one of the preceding claims, wherein the locking device (27, 29) comprises a toggle joint, wherein the clamp (14) is hinged in rotation about a first axis of rotation relative to the body (13), and wherein the jaw (11) comprises a connecting rod (27) hinged in rotation with the clamp (14) about a second axis of rotation (28), said second axis of rotation (28) being parallel with the first axis of rotation (15), wherein the jaw (11) comprises at least one rocker element (29) hinged in rotation with the connecting rod (27) about a third axis of rotation (30), on the one hand, and with the body (13) of the jaw (11) about a fourth axis of rotation (31), on the other hand, the third and fourth axes of rotation (30, 31) being parallel to each other and with the first and second axes of rotation (15, 28), and wherein the rocker element (29) and the connecting rod (27) together form the toggle joint, wherein the toggle joint is configured to be displaced between an unlocked configuration in which the third axis of rotation (30) is located on a first side of the plane (P) formed by the second and fourth axes of rotation (28, 31) and the jaw (11) is in the open position, and a locked configuration in which the third axis of rotation (30) is located on a second side of said plane (P) and the jaw (11) is in the closed position, wherein the jaw (11) comprises a blocking device (39, 40) configured to block the toggle joint (27, 29) in the locked configuration, wherein the blocking device (39, 40) comprises
- a stop (39) of the connecting rod (27) configured to come into contact with the rocker element (s) (29), when the toggle joint (27, 29) is in the locked configuration, or
- a protrusion (40) of the clamp (14) configured to come into contact with the connecting rod (27), when the toggle joint (27, 29) is in the locked configuration.

9. The implant (10) according to one of the preceding claims, wherein the body extends along a longitudinal axis (X), the clamp (14) is hinged in rotation relative to the body (13) about a first axis of rotation perpendicular to the longitudinal axis (X), and the pusher element (41) is configured to be displaced in translation along the longitudinal axis (X) relative to the body (13) of the jaw (11), and wherein the displacement device further comprises a lever element (42) hinged in rotation relative to the connecting rod (27) about the third axis of rotation (30), on the one hand, and relative to the pusher element (41) about a fifth axis of rotation (43) parallel with the first axis of rotation (15, 28, 30, 31),on the other hand, wherein the body (13) and the clamp (14) each comprise an engagement portion (32, 33) which are arranged opposite to each other and configured to enclose the first leaflet of the heart valve, for example the posterior leaflet (FP) of a mitral valve (VM), when the jaw (11) is in the closed position, and wherein the pusher element (41) is further configured to be displaced in translation along the longitudinal axis (X) by sliding against a face (44) of the body (13), opposite to the engagement portion (33) of the clamp (14).

10. The implant (10) according to one of the preceding claims, wherein the body extends along a longitudinal axis (X), and wherein the pusher element (41) is provided with an opening (45) extending along the longitudinal axis (X) and configured to cooperate with the distal end of the rod (101), wherein the body (13) comprises a guide element (46) configured to guide a translation of the distal end of the rod (101) along the longitudinal axis (X).

11. The implant (10) according to claim 10, wherein the guide element (46) extends from the face (44) of the body (13) opposite the engagement portion (33) of the clamp (14) and comprises an opening (47) coaxial with and opposite to the opening (45) of the pusher element (40), said opening (47) of the guide element (46) being configured to slidably receive the distal end of the rod (101) .

12. The implant (10) according to one of the preceding claims, wherein the body (13) and the clamp (14) each comprise an engagement portion (32, 33) which are arranged opposite to each other and configured to enclose the first leaflet of the heart valve, for example the posterior leaflet (FP) of a mitral valve (VM), when the jaw (11) is in the closed position, and wherein the engagement portion (32, 33) of the body (13) or the clamp (14) comprises a plurality of tips (34) configured to stick into the first leaflet when the jaw (11) encloses the first leaflet.

13. The implant (10) according to the preceding claim, wherein at least one opening (35) is formed in the engagement portion (33, 32) of the clamp (14) or of the body (13), opposite to the tips (34) of the body (13) .

14. A set comprising:
- an implant (10) for a heart valve according to one of the preceding claims,
- a rod (101) whose distal end is configured to cooperate with the pusher element of the implant (10), so as to open and close said jaw (11).

15. The set according to the preceding claim, further comprising a main catheter (100) configured to receive the implant (10) and the rod (101).
